# EUROPEAN PATENT SPECIFICATION

(11) **EP 1 689 327 B1**
(45) Date of publication and mention of the grant of the patent: **14.09.2016**
(21) Application number: 04819616.6
(22) Date of filing: 24.11.2004
(51) Int. Cl.: A61F 2/95

(54) **CATHETER DEVICE**
KATHETER
CATHETER

(30) Priority: 24.11.2003 GB 0327306
(43) Date of publication of application: 16.08.2006
(73) Proprietor: Angiomed GmbH & Co. Medizintechnik KG, 76227 Karlsruhe (DE)
(72) Inventor: DORN, Jürgen, 68809 Neulussheim (DE)
(74) Representative: Hoffmann Eitle
(86) International application number: PCT/EP2004/013339
(87) International publication number: WO 2005/053574

(56) References cited:
- WO-A-03/002019
- US-A1- 2003 109 886

## Description

### Background of the Invention

### Field of the invention

This invention relates to a catheter device having a shaft, a rapid-exchange guidewire lumen (one which terminates at a proximal guidewire exit port that is distal of the proximal end of the catheter) and a distal end which exhibits a sheath which can be withdrawn proximally to release a self-expanding implant such as a stent. To prevent the self-expanding stent moving proximally with the proximally-moving sheath, the catheter device includes a stopper which bears on the stent and resists its proximal movement.

Conventionally, such a catheter device exhibits a shaft comprising an outer tube connected to the sheath and an inner shaft connected to the stopper, whereby the proximal movement of the sheath is accomplished by imposing an endwise tension on the outer tube, with the inner shaft carrying an endwise compression stress, as the stopper at the distal end of the inner shaft works to resist proximal movement of the stent. For examples, see WO 03/003944, WO 03/002020, WO2004/062458, US-A1-2003/0109886 and EP-A-1095634. The features of the precharacterizing part of claim 1 below are disclosed, inter aliea, in US-A1-2003/0109886.

Such conventional systems can work well, and can be of relatively simple construction. However, the present inventor has discovered that they are nevertheless capable of improvement.

One disadvantage noted by the present inventor is that release of the stent requires the medical practitioner to maintain the inner pusher shaft unchanged in axial disposition relative to the site of stenting in the body of the patient, while pulling back on the outer tube of the shaft to release the stent. This pulling back of the outer tube requires relative movement of the outer tube in the bodily lumen (or guide catheter) in which it has been advanced to the site of stenting. Any friction or resistance to axial movement of the outer tube in the lumen in which it is located hinders the objective of maintaining the stopper in a precise disposition relative to the target stenting site. In practice, it is customary to compensate for axial strain in known systems by positioning the stent slightly distal of the desired end position before commencing stent deployment by pulling back the sleeve. The present invention aims to reduce or eliminate the need for such compensation.

### Summary of the Invention

The invention is defined in claim 1 below. The dependent claims are directed to optional features and particular embodiments. Furthermore, insolar as the terms "invention" and/or "embodiment" are used in the following, and/or features are presented as being optional, this should be interpreted in such a way that the only protection sought is that of the invention as claimed.

One object of the present invention is to improve positional placement of a self-expanding stent at a target stenting site in a human or animal body, when using a transluminal, catheter-based stent delivery system. According to one aspect of the present invention, there is provided a catheter device of the type identified above, and which is characterised in that the shaft of the catheter device features a shaft pusher tube with a lumen and with a distal end operatively connected to the stent stopper, the lumen of the pusher tube being occupied by a pull wire or rod which is arranged to pull back the sheath surrounding the self-expanding stent. The wire or rod can itself be tubular. It is resistant to endwise extension of its length, and the pusher tube is resistant to endwise shortening of length when placed in endwise compression. Normally, both so-axial elements will be of a suitable metal such as stainless steel.

The present invention is useful in a method of deploying a self-expanding stent in which a sheath surrounding the stent is pulled back proximally by a pull wire within the shaft of a rapid-exchange transluminal catheter delivery system for the stent.

It will be appreciated that, with an arrangement in accordance with the present invention, there is no requirement for any axial movement of the outer shaft tube relative to the lumen in which it slides. Instead, since the shaft tube is connected to the stent stopper, it is required that there be no such axial movement. Accordingly, any binding between the shaft tube and any surrounding guide catheter, or bodily tissue of the access lumen, and any friction acting on the outside surface of the shaft tube, is turned by the present invention into an advantage rather than a problem, because it will help to confirm the axial position of the shaft tube relative to the stopper and the stenting site. The more tortuous the access lumen in the body, the more likely it is that there will be no axial movement of the shaft tube and stopper relative to the intended stenting site.

Furthermore, a shaft tube has more inherent resistance to elastic axial compression than a mere wire within the lumen of the tube. Thus, regardless how great are the tensile stresses imposed on the pull wire during the push-pull activity of stent release, there should be less unwanted proximal movement of the stopper from the intended site of stenting. In preferred embodiments of the invention, the shaft tube is of stainless steel or of a cobalt/chromium/nickel alloy sold under the trademark PHYNOX.

Furthermore, the sheath itself can also be metal-reinforced (such as by an embedded metal braid) and so also with a high capacity to resist axial strain, increasing the precision with which the operator of the catheter device can control the progressive withdrawal of the sheath and release of the stent. Many doctors prefer to release a self-expanding stent in a step-wise movement. If the pulling system stretches, then a step-wise movement can have the consequence of a time-dependent response at the distal end of the system, and a relaxation of the pulling system between successive pulling steps, with consequent undesirable reverse distal movement of the sheath or else "lost movement" in the pulling system as it once again strains to take up the pull tension with successive step-wise pulls at the proximal end of the system.

Thus, the shaft tube is conveniently a stainless steel or PHYNOX hypo tube and the pull wire is conveniently of metal, such as a stainless steel wire, either solid or hollow. While the sheath will very likely be of polymer, it can be made resistant to elastic stretching during proximal withdrawal and release of the stent by embedding within the annular wall thickness of the polymer sheath a fiber reinforcement such as a braided metal mesh. In such an embodiment, there is effectively a continuous strand of elastic strain-resistant metal in the pulling system, all the way from the proximal end of the pull wire to the distal end of the polymer sheath, again adding to the precision of proximal withdrawal, and minimising any elastic strain within the system during withdrawal.

The pull wire can be connected to the sheath by, for example, first and second metal rings, one inside the other, and sandwiching the sheath so that one of the metal rings is inside the sheath annulus and the other is outside the sheath annulus. The inside metal ring would normally be welded, soldered or brazed to the distal end of the pull wire (adhesives being generally disfavoured in failure-critical applications in such stent delivery devices) while the outer metal ring can be swaged down onto the sheath to press the sheath onto the inner metal ring.

The present applicant has developed stent delivery systems (see WO2001/34061) which feature a catheter system having a heat-formed tapered distal tip which can help to reduce trauma to the body as the catheter system is advanced in a bodily lumen along its guidewire. In the present invention, it is preferred that the sheath has a tapered distal tip, which can be heat-formed, and which desirably tapers down to an end orifice which fits relatively closely around the cylindrical outside surface of the guidewire.

In some embodiments, the catheter shaft diameter is defined by the pusher tube, and is smaller than the diameter of the sheath around the stent. At the proximal end of the sheath, in such embodiments, it may be attractive to taper the diameter down to a relatively snug fit around the outside of the shaft tube (but not so snug as to resist proximal axial sliding of the sheath along the outside of the shaft tube). It is contemplated to create the proximal guidewire exit port in the tapered proximal end of such a formed sheath, as explained below in more detail in relation to the accompanying drawings.

In another embodiment, the proximal end of the sheath can be joined to a metal collar that defines a proximal guidewire exit port lumen and another lumen to slidably receive the outer tube of the catheter shaft. The collar can be given a domed shape facing proximally, to facilitate atraumatic withdrawal of the catheter system.

One way of connecting the shaft tube to the stopper is by way of a pusher-guider tube which defines a guidewire lumen and carries the stopper at a location near the distal end of the pusher-guider tube, or at its distal end. The proximal end of the pusher-guider tube is arranged to one side of the distal end of the shaft tube and fixed relative to it, such as by welding or glueing. Conveniently, both the pusher-guider tube and the shaft tube are of metal such as stainless steel, simplifying the task of bonding together side-by-side the proximal end of the pusher tube and the distal end of the shaft tube, as by welding or brazing. Other means of joining these tube sections will be apparent to those readers skilled in the field, who will also appreciate that adhesive compositions are generally disfavoured, whenever failure of the adhesive bond results in failure of the device and risk to the patient, in use.

Distal of the stopper, the pusher-guider tube in preferred embodiments is not required to carry any substantial axial compressive stress. In any event, it should be soft and easily bendable so as to keep the catheter tip as floppy as possible. The compression resistant pusher-guider tube could be extended distally beyond the stopper, all the way to the distal end of the sheath, in order to define a guidewire lumen which extends within the pusher tube all the way to the distal end of the system. Indeed, the pusher tube could extend into an atraumatic tip distal of the distal end of the sheath. In this way, the tapered tip of the sheath could be omitted.

Thus, there can be provided, distal of the stopper, a pusher tube extension, which continues the guidewire lumen from the stopper to the distal end of the system, but which may be of less heavy construction, being formed for example of thin wall polymer tube. Another useful purpose of such a guidewire lumen distal of the stopper is for carrying a radiopaque marker band to indicate the distal end of the stent within the delivery system, so that the radiologist can determine with precision where the stent in the delivery system is located relative to the target stenting site.

For the sake of completeness, and to put the present invention in the context of the prior art documents seen with hindsight to be helpful in appreciating how the present invention contributes to the state of the art, reference will now be made to EP-A-611 556 and WO 96/39998.

EP-A-611 556 discloses a rapid exchange balloon catheter stent delivery system in which a sheath is pulled back proximally by a pull wire, to expose a stent mounted on a balloon, so that the stent can then be deployed by inflation of the balloon. The stent is not a self-expanding stent, so is not pressing on the luminal surface of the sheath during advance of the delivery system to bring the stent into the location of stenting. Accordingly, the balloon-expandable stent is not liable to be carried proximally by the sheath when the sheath is pulled proximally. Accordingly, there is no need for a stopper to resist unwanted proximal movement of the stent. Accordingly, there is no significant resistance to proximal movement of the sheath. Accordingly, there is no need for the shaft of the system, defining the lumen in which the pull wire is located, to be resistant to axial compressive stresses. The problem of designing a system to deliver a self-expanding stent which maintains the axial position of the stent correct during stepwise release of the stent is not a problem experienced with balloon-expandable stent delivery systems.

Conversely, WO 96/39998 is a disclosure which is concerned with systems which will resist endwise compression during delivery of a self-expanding stent and proximal withdrawal of a sheath surrounding such a stent. The problem is addressed by providing within the delivery system an inner core which is resistant to endwise compression, and providing a stopper near the distal end of the inner core. Thus, the pull wire is not housed within the lumen of the element that is in endwise compression during stent release but instead, is lying side-by-side with the element that is subject to endwise compression. Any capability that the outer sheath of the system might have to carry endwise compression stress remains unutilised.

For a better understanding of the present invention, and to show more clearly how the same may be carried into effect, reference will now be made, by way of example, to the accompanying drawings.

### Brief Description of the Drawings

- Fig. 1: is a longitudinal diametrical section through the distal end zone of a catheter device in accordance with the invention;
- Fig. 2: is the identical section, at larger scale, through the distal part of the distal zone of Fig. 1;
- Fig. 3: is an identical section, at larger scale, through the proximal part of the distal zone of Fig. 1; and
- Fig. 4: is a longitudinal diametrical section, at enlarged scale, of the junction between the pusher tube and pusher tube extension of the embodiment of Fig. 1.
- Fig. 5: is a view from the side of a second embodiment of the invention
- Fig. 6: is a longitudinal diametral section through the distal end of the second embodiment
- Fig. 7: is an isometric view of the adaptor block of Fig. 6
- Fig. 8: is a longitudinal diametral section through a shaft portion of the second embodiment, including a guider block
- Fig. 9: is an isometric view of the guider block of Fig. 8
- Fig. 10: is a transverse section through the guider block, on the line X-X in Fig. 8
- Fig. 11: is a longitudinal diametral section through the proximal part of the shaft of the second embodiment
- Figs. 12 and 13: are sections through two alternative proximal ends of the pull wire of the second embodiment, and
- Fig. 14: is a longitudinal medial section through the hand unit of the second embodiment.

### Detailed Description

### First Embodiment

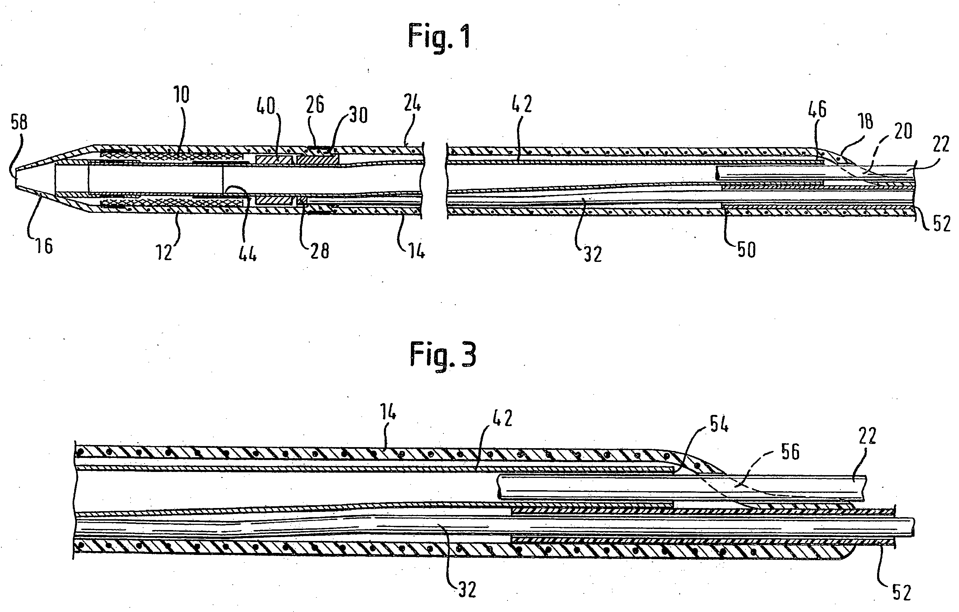

Referring to Figs. 1, 2 and 3, a self-expanding stent 10, or stent graft, lies inside the distal end zone 12 of a sheath 14 with a tapered distal tip 16 and a heat-formed proximal end 18 which defines the orifice 20 of a proximal guidewire exit port for a guidewire 22. Being a self-expander, the stent 10 is, at least at body temperature, putting compressive stress on the luminal surface of the sleeve 14 in the distal end zone 12. Proximal of the stent 10, and on the abluminal surface 24 of the sleeve 14, is a swaged marker band 26 of radiopaque metallic material, which is pressing radially inwardly the material of the sheath 14 within the band 26. Radially inside the sheath at this point is a stepped metal annulus 28 which is itself put under radially inwardly compressive stress by the material 30 of the sheath 14 inside the marker band 26. Thus, the sheath material 30 is compressed between metal bands inside (28) and outside (26) the sheath 14. Brazed to the annulus 28 is a pull wire 32 which runs from the annulus 28 all the way back to the proximal end of the catheter device, whereby endwise tensile stress imposed on the proximal end of the pull wire 32 will pull proximally the annulus 28 and thereby impose on portions of the sheath 14 distal of the annulus 28 an endwise tensile stress, for pulling the sheath 14 proximally with respect to the stent, to release the stent. At the same time, portions of the sheath 14 proximal of the annulus 28 will be pushed proximally.

A pusher annulus 40 is located in the lumen of the sheath 14 just proximal of the stent 10. Its purpose is to resist proximal movement of the stent 10, when the sheath 14 is withdrawn proximally from the stent 10. It can also serve as a radiopaque marker band to indicate the proximal end of the stent 10. The pusher annulus 40 is brazed or welded or otherwise fixed to a pusher-guider tube 42 which is conveniently of stainless steel or PHYNOX™ and which has its distal end 44 distal of the pusher annulus 40 and within the lumen of the stent 10. The proximal end 46 of the pusher tube 42 is arranged side-by-side with the distal end 50 of a shaft pusher tube 52 of the catheter device which extends all the way to the proximal end of the catheter device and is conveniently provided as a PHYNOX or stainless steel hypo tube. The lumen of this shaft tube 52 carries the pull wire 32. The overlapping portions 46 and 50 of the pusher-guider tube and shaft pusher tube are bonded to each other, conveniently by brazing, so that they effectively form a single metal strand from the proximal end of the catheter device to the stent pusher annulus 40. As can be seen in Fig. 1 and Fig. 3, the end orifice 54 of the pusher tube 42 is colinear with the orifice 56 in the heat-formed end 18 of the sheath 14, which defines the proximal guidewire exit lumen. Thus, when a guidewire 22 is advanced through the guidewire lumen of the catheter device by introducing it into the end orifice 58 of the tapered distal tip 16 of the sheath 14, the end of the guidewire will advance proximally along the pusher tube and exit through the port 56.

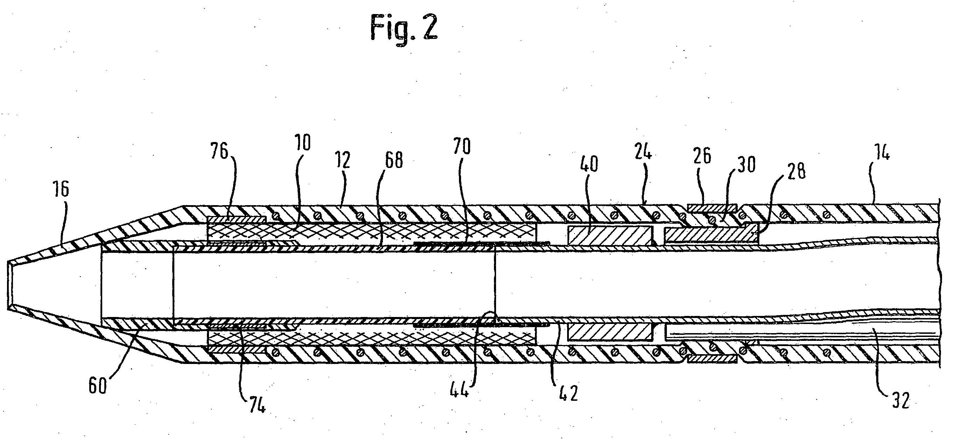

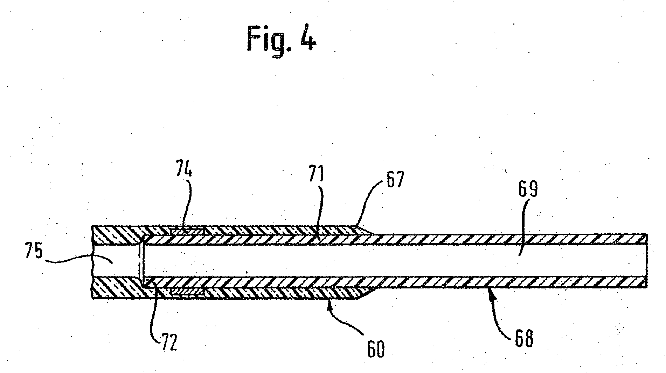

With reference to Fig. 2 and Fig. 4, we will now explain the structure of the pusher tube extension, distal of the pusher annulus 40, and located between that annulus and the end orifice 58 at the distal end of the sheath 14.

The metal pusher tube 42 extends for a short distance distally of the pusher annulus 40. A distal extension inner catheter 68 of polyimide abuts the distal end of the pusher tube 42 and is secured to that pusher tube by a shrink tube 70 radially overlying the distal end of the pusher tube 42 and the proximal end of the inner catheter 68. This shrink tube 70 is of PET (which shrinks radially downward to grip both these abutting portions).

Fig. 4 shows the distal end 72 of the distal extension inner catheter tube 68 and a bore 69 within it, open to the distal end of the inner catheter 68, and terminating proximally at an end-to-end butt joint with the distal end of the metal pusher tube 42. A tip extension catheter 60 of PEBA polymer (PEBAX®) receives the distal end 72 of the inner catheter 68, so that its proximal end 67 overlaps the abluminal wall of the catheter 68. Around the distal end 72 of the catheter 68, and sandwiched between the distal catheter 68 and the proximal end zone of the tip catheter 60, is a second radiopaque metal marker band 74, and the whole assembly is bonded together with a cyanoacrylate adhesive composition. The PEBAX tip extension catheter 60 extends into the tapered lumen of the taper 16 of the distal end of the sheath 14.

Of note is that the bore 75 of the catheter 60 is contiguous and smooth with the bore 69 of the catheter 68 for smooth progress of a guidewire. Catheter 60 is soft and floppy but has a larger outside diameter than catheter 68, which helps to ease the end orifice of the sheath 14 open when it begins to withdraw. Proximal end 67 of catheter 60 is tapered inwardly. This is because, should a physician decide to sheath the distal end of the delivery system after stent deployment by re-advancing the sheath distally, the tapered tip 16 of the sheath is required to advance distally back onto the abluminal surface of catheter 60 and the taper 67 helps that advance.

Reverting to Fig. 2, fixed to the lumen surface of the sheath 14, just proximal of the tapered tip zone 16, is a third radiopaque metal marker band 76 and it will be seen that this marker band lies radially outside the second marker band 74 within the distal extension inner catheter 68.

To deploy the stent the pull wire is pulled y an actuator at the proximal end of the system. A suitable actuator is described below, as part of a second embodiment illustrated herein.

In use, the distal end zone of the catheter system, as shown in the drawings, is advanced along a bodily lumen to a stenting site. When all is ready for deployment of the stent 10, an endwise tension is applied to the pull wire 32, while the proximal end of the shaft tube 52 is restrained from endwise movement, reactive or otherwise. Endwise translation of the pull wire 32 results in proximal movement of the sheath 14. Holding the endwise position of the shaft tube 52 holds the endwise position of the pusher annulus 40 which in turn prevents any proximal movement of the stent 10 with the proximally withdrawing sheath 14.

Progressively, the sheath 14 withdraws proximally relative to the stent 10, having the effect of stretching the distal tip 16 of the sheath 14 over the radially outward surface of the stent 10, leading to progressive release and radial expansion of the stent 10, from its distal end toward its proximal end.

Note that, before there is any relative movement of the sheath 14 and pusher annulus 40, the radiologist "sees" only two marker bands, namely the first marker 40 and the radially superimposed second and third marker bands 74 and 76. However, once the sheath 14 starts to withdraw proximally, the radiologist can see the third marker, at a position proximal of the second marker. Clearly, when the third marker has moved proximally to approach, pass over, and then move proximally away from the first marker 40, one has confirmation that the stent 10 has been deployed, by full proximal withdrawal of the sheath 14.

During proximal withdrawal of the sheath 14, it will be appreciated that the proximal end 18 of the sheath 14 slides proximally over the outside surface of the shaft tube 52.

It will appreciated that there should be no endwise movement of the shaft 52 relative to its surrounding entities, whether a bodily lumen or the lumen of a guide catheter, during deployment of the stent 10. This is an opportunity for enhancement of precision of the placement of the stent, because any friction between the outside surfaces of the shaft tube 52 and the surrounding structures will only tend to confirm the location of the pusher annulus with respect to the body of the patient, and thereby the location of the stent 10 with respect to the body of the patient.

Further, the friction forces between the pull wire 32 and the luminal surfaces of the shaft tube 52 ought to be very small or minimal, as should any frictional forces between the withdrawing sheath 14 and the outside surface of the shaft tube 52, at the proximal end 18 of the sheath. Further, as the sheath 14 is relatively short in proportion to the catheter device as a whole, any friction between the outside surfaces of the sheath 14 and the surrounding bodily tissue ought also to be usefully smaller than in conventional systems where the full length of the stent deployment catheter must be moved relative to its surroundings. All of this elimination of unwanted and unhelpful friction is advantageous to the person deploying the stent, because any tactile feedback should relate more closely to events at the stent itself, and any force input at the proximal end of the device should be more completely delivered to the components around the stent 10 at the distal end of the device. There should be less lost motion in the system between the proximal and distal ends, less hysteresis, and less discrepancy between the amount of force applied at the proximal end and the amount of force delivered to the components surrounding the stent. It should be possible, with the system proposed herein, to enhance the position of stent placement, and the degree of confidence that users have when deploying stents, that the stent has been deployed smoothly and correctly.

As to design variations, the following will be evident to those skilled in the art, but so too will many more design possibilities, within the relevant published state of the art but not mentioned here.

The sheath need not include braiding. The pull wire can be threaded directly to the braiding, thereby avoiding the need for any pulling annulus between the pull wire and the sheath. Neither the distal end nor the proximal end or the sheath need be tapered. An atraumatic tip to the device can be carried on the pusher sub-system that includes the stent stopper.

Implants to be delivered by the device need not be stents and stent graft. For example, filters can be deployed with the device.

Those skilled in the art will appreciate how to build an actuator for the proximal end of the device. A suitable basis is the device described in WO 02/087470, modified to accommodate the radial inversion of the push/pull elements.

### Second Embodiment

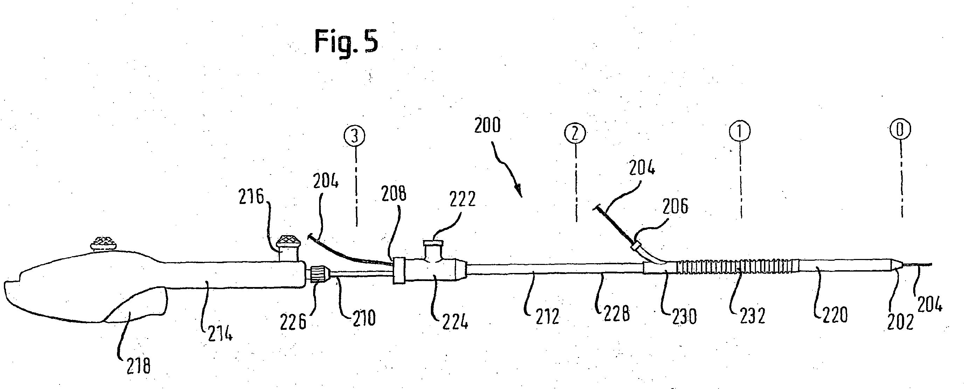

Fig. 5 is a general view from the side of a second embodiment of the invention, in which a delivery system 200 for a self-expanding stent 201 has a distal end 202 that advances over a guidewire 204, with the guidewire proximal end advancing proximally along a guidewire lumen 203 as far as a chosen one of two alternative guidewire exit ports 206, 208 both distal of the proximal end 210 of the shaft of the catheter 212 of the system. A hand unit 214 includes a first actuator 216 and second actuator 218 that can be used alternatively or sequentially to pull proximally a sheath 220 that radially overlies the self-expanding stent at the distal end of the system. At a port 222 in a proximal hub 224, flushing liquid can be introduced, to flush the system of air bubbles. A swivel nut 226 connects the catheter shaft 212 to the hand unit 214 to allow the hand unit to rotate freely around the long axis of the shaft.

Distal of the hub 224, the catheter shaft is defined by a flushing sleeve 228, which extends distally to a guider block 230 which defines the more distal of the two alternative guidewire exit ports 206. Distal of the block 230 and proximal of the stent sheath 220 is a PET bellows sleeve 232 that is contiguous with both the flushing sleeve 228 and the stent sheath 220. As the stent sheath 220 is pulled proximally, it gets closer to the adaptor 230, and the bellows sleeve 232 can undergo a reduction in length to accommodate this proximal movement. An adhesive such as DYMAX® is used to secure the bellows sleeve to the flushing sleeve.

The guidewire port is conveniently located away from both ends of the catheter system. possibly about half way along the length, or around 75 cm from the distal tip of the system.

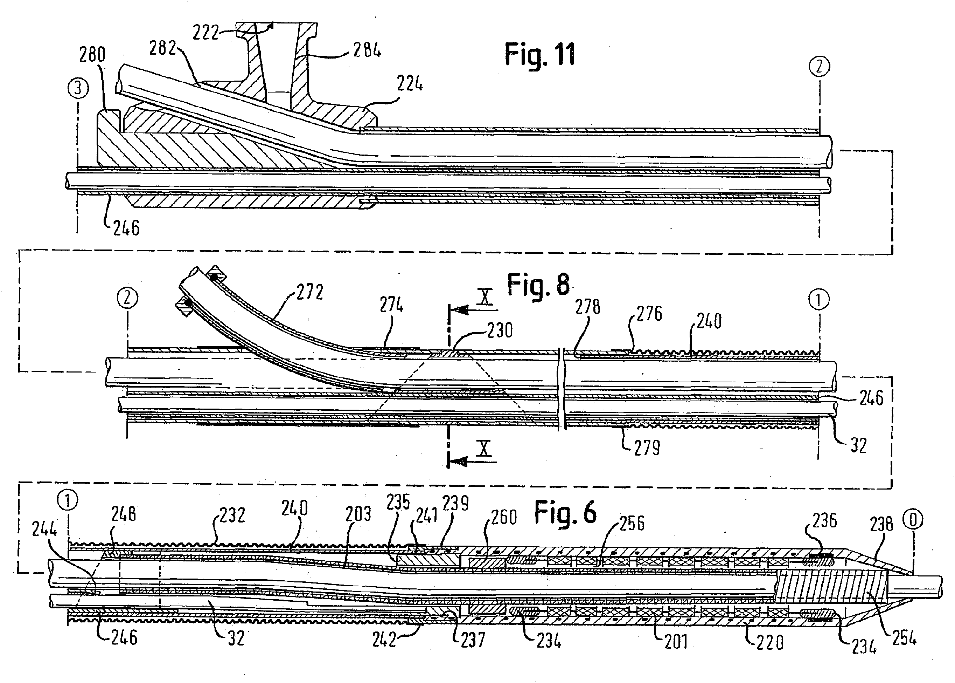

Turning to Fig. 6, we see that the stent is provided at each end with a ring of tantalum marker spoons 234 (see Applicant's WO2002/015820). The sheath 220 confining the stent is of PEBA polymer (PEBAX®) reinforced with a braid of flat stainless steel wire at 45 or 65 pitches per inch, 0.13 or 0.075 mm wide and 0.025 mm thick and it has a liner of PTFE (TEFLON®). The sheath has a platinum/iridium marker band 236 embedded within it and overlying the distal end of the stent. Distal of the marker band is a tapered distal tip 238 made of a softer grade of PEBA. The proximal end of the sheath is surrounded by a stainless steel stepped swaged band 239 that presses the sheath 220 inwardly down over a shoulder 237 on a stainless steel pull ring 235 that is welded to the stainless steel, PTFE-coated pull wire 32. The swaged band also presses onto the pull ring the distal end 241 of a telescope tube 240, and shrunk down onto the radially outer surface of the swaged band is the distal end 242 of the bellows sleeve 232.

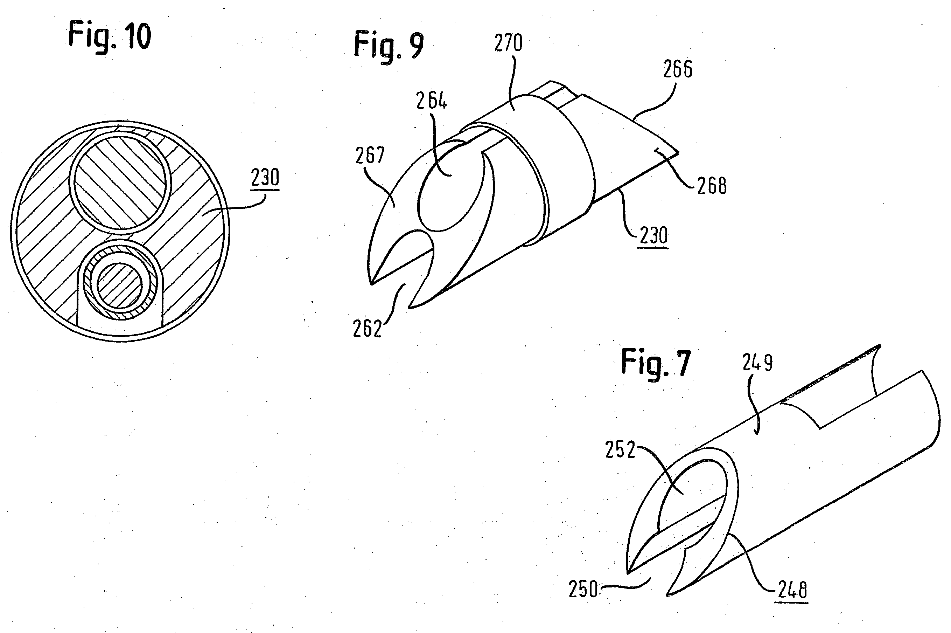

The pull wire enters the distal end 244 of a PHYNOX pusher tube 246, to which is glued (DYMAX®) a PEBA pusher adapter block 248, Fig. 7, which defines two lumens side-by-side, one (250) for the pusher tube and the other (252) for a pusher guider tube 254 which defines the guidewire lumen 203. The pusher-guider tube is made of a closed turn spiral of flat stainless steel wire 0.09 mm thick and 0.25 mm wide with a lumen diameter of 0.95 mm. The outside diameter is ground down to 1.07 mm for the section of its length that lies within the stent lumen 256. The pusher-guider tube is co-axial with the catheter shaft from the distal tip 238 of the system until the pull ring 235. Just distal of the pull ring, the spiral carries on its radially outside surface a pusher ring 260 which faces the ring 234 of spoons at the proximal end of the stent. The pusher ring can be a simple stainless steel ring, or a composite of a stainless steel ring proximally adjacent a ring of polymer such as PEEK to abut the stent. The outside diameter of the pusher ring in this embodiment is 2 mm, that is, 6 French.

Proximal of the pull ring, the pusher-guider tube veers through a gentle double bend to resume a straight line axial course parallel to the pull wire, and as far as the adapter block 248 to which it is fixed in the block lumen 252 with an adhesive (DYMAX®).

Looking now at Fig. 8, the telescope tube 240 is of polyimide, adds stiffness to the portion of the shaft length that it occupies, and slides over the outside cylindrical surface 249 of the adapter block 248 in a proximal direction, when the stent sheath is pulled proximally to release the stent. The telescope tube in its proximal movement approaches (but does not abut) the PEBA guider block 230 that is mounted (e.g. by DYMAX® glue) to the pusher tube 246. It is located in this embodiment about 75 cm from the distal tip of the catheter. The guider block, see Figs. 9 and 10, defines two lumens side-by-side, one (262) for the pusher tube and the other (264) for the guidewire. It has a chamfered distal face 266 that helps to steer the proximal end of the guidewire into the lumen during back-loading of the guidewire. Analogously, its proximal-facing end wall 267 is also inclined. It carries on its outside cylindrical surface 268 the flushing sleeve 228 and over that sleeve is a PEBA band 270 that squeezes the sleeve 228 onto the block surface 268, and the block material each side of lumen 262 onto the pusher tube within lumen 262. Just proximal of the block is an aperture (not visible) in the flushing sleeve 228 that receives a polyimide guidewire steering tube 272 for leading the guidewire out of its lumen in the catheter shaft at the distal exit port 206. If the user chooses not to use this exit port, the steering tube 272 is simply pulled away from the system. The side hole left behind in the wall of the flushing sleeve is closed by a thin PEBA shrink sleeve 274 that overlies radially the flushing sleeve where the steering tube exit hole is found. Absent the steering tube, the guidewire may continue to advance proximally within the flushing sleeve and alongside the pusher tube 246. The flushing sleeve has a distal end somewhat distal of the guider block 230. It receives within its distal end opening 276, telescopically, the proximal end 278 of the telescope tube 240. It is of polyimide. The bellows sleeve is glued to the flushing sleeve at an overlap 279, as mentioned above.

In a first variant, the telescope tube could be radially outside the flushing sleeve.

In a second variant, the flushing sleeve can be integral with the bellows sleeve, thereby obviating the need for any telescopic arrangement.

Following proximally the shaft of the system to the more proximal of the two alternative guidewire exit ports brings us to Fig. 11 in which we see the flushing sleeve ending proximally in a polyamide hub 224 through which the pusher tube 246 continues on proximally. A stainless steel wedge piece 280 is provided in a guidewire lumen 282 of the hub to steer the proximal end of the guidewire through the hub and out of its exit port 208. Communication with the guidewire lumen and flushing sleeve lumen is the flushing port 222 with female Luer lock connector portion 284.

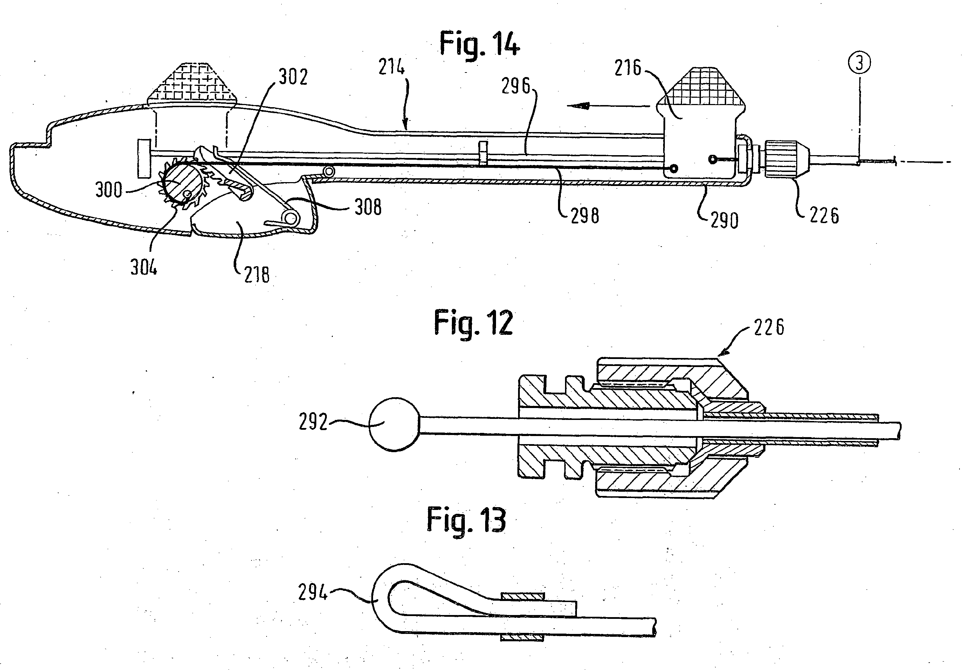

Moving on to Figs. 12, 13 and 14, the pusher tube ends proximally in the polyamide swivel nut 226 that is mounted to the housing 290 of the hand unit 214. Thus, axial movements of the housing relative to the bodily lumen in which the delivery system lies will be transmitted from the housing to the pusher ring 260 via the push tube 246, to move the stent axially within that lumen. The pull wire is attached by a brass nipple 292 (Fig. 12) or by a loop 294 (Fig. 13) to a polyamide slider 216 (Fig. 14) which is the first actuator of the hand unit. The slider 216 runs on a pair of stainless steel rails 296 mounted to the housing, whereby pulling the slider on the rails pulls the pull wire relative to the swivel nut thereby pulling the stent sheath proximally.

But the slider 216 is on the distal end of a pulling line 298 which is wound up on a drum 300 journalled in the housing. Each pump on a trigger 218 causes a toothed rack piece 302 to advance in engagement with the teeth of a toothed wheel 304 on the drum, and a pawl stops any reverse movement of the toothed wheel and drum during return movement of the rack 302 and trigger 218 after each squeeze of the trigger. The return movement is induced by a return spring 308, the bias of which has to be overcome during each squeeze of the trigger. Thus, the stent can be released by a succession of squeezes on the trigger, or by one long smooth proximal stroke of the slider, or by any combination of these two actuators (see Applicant's earlier WO2002/087470).

It will be appreciated that the illustrated embodiments, and the invention as claimed, make available a system to deploy a self-expanding stent, or other implant, that has a number of valuable advantages, including:
i) no axial movement of the outer surface of the shaft of the delivery system relative to surrounding bodily tissue during stent deployment
ii) long thin load-bearing components entirely of metal, for both co-axial parts of the stent release system, so minimising length changes when the shaft length is suffering the endwise stresses that are imposed on it when the stent sheath is being pulled proximally off the stent
iii) the tolerance of different stent lengths and diameters that flows from a design that is inherently modular (see Applicant's WO2003/003944)
iv) choice of two different lengths of guidewire lumen
v) an absence of re-entrant surfaces on the tip of the system inside the stent lumen, so that withdrawal of the system after deployment of the stent should not carry the risk of dislodging or parting bodily tissue from the stenting site as the tip withdraws proximally through the stent lumen (see Applicant's WO2001/034061).

The system illustrated in Figs. 5 to 14 has been described with diameter dimensions. It will be appreciated that these dimensions can all be modified, more or less in proportion, to create other systems with a range of different overall diameters.

A number of published documents have been mentioned above. Many of these are from Applicant, and represent steps along the way to the present invention.

## Claims

1. A catheter device having a shaft that extends from a proximal end to a distal end to carry on its distal end a self-expanding implant (10) for intraluminal advance on a guidewire (22) and delivery of the implant to a stenting site by proximal withdrawal of a sheath (14) that lies radially outside the implant in the catheter, the catheter including a first shaft element (32) to pull the sheath proximally and a second shaft element (42, 52) to push the implant distally to prevent the implant moving proximally with the sheath when the sheath is pulled proximally, wherein
i) the shaft defines a flushing lumen
ii) the shaft defines a proximal guidewire exit port (20) that is distal of the proximal end of the shaft, for rapid exchange of the catheter with respect to the guidewire, and **characterized in that**:
iii) the first shaft element is a pull wire
iv) the second shaft element is a pusher tube (16).

2. Catheter as claimed in claim 1, wherein the sheath has a tapered distal tip (16).

3. Catheter as claimed in claim 1 or 2, wherein the pull wire is coupled to the sheath by an inner pull ring (28) located radially inside the sheath, the pull wire and pull ring being both of metal and with a metal bond between the pull wire and the pull ring.

4. Catheter as claimed in claim 3, including an outer pull ring (26) radially outside the sheath and the inner pull ring, with the sheath compressed between the inner and outer pull rings.

5. Catheter as claimed in any one of the preceding claims, wherein the sheath is polymeric and carries within its wall thickness a braid of metallic filaments.

6. Catheter as claimed in any one.of the preceding claims wherein the pusher tube (52) has a distal end to which is fixed side-by-side, the proximal end of a pusher-guider tube (42) that defines a lumen for said guidewire.

7. Catheter as claimed in claim 6, wherein the pusher-guider tube (42) is formed from a spiral metal filament and carries a stopper ring (40), the pusher tube, pusher-guider tube and stopper ring serving as said second shaft elements.

8. Catheter as claimed in claim 7, wherein the pusher-guider tube extends distal of the stopper ring, to the distal tip of the catheter.

9. Catheter as claimed in claim 6, 7 or 8, with an adaptor block (248) to connect the pusher tube and the pusher-guider tube, the block defining two lumens side-by-side, one for the pusher tube and the other for the pusher-guider tube.

10. Catheter as claimed in any one of the preceding claims, including a flushing sleeve (228) that is contiguous with the sheath and defines a lumen that contains the pusher tube.

11. Catheter as claimed in claim 10, wherein the shaft includes a guider block (230) that has a cylindrical outside surface to receive the flushing sleeve, a guidewire lumen, and a lumen side-by-side with the guidewire lumen to receive the pusher tube.

12. Catheter as claimed in claim 11, wherein the flushing sleeve distal of the guider block includes a bellows tube (232).

13. Catheter as claimed in claim 11 or 12, wherein the flushing sleeve distal of the guider block includes a telescopic tube.

14. Catheter as claimed in claim 11, 12 or 13, wherein the guider block is fixed against axial movement with respect to the pusher tube and with respect to the flushing sleeve on its cylindrical surface.

15. Catheter as claimed in claim 14, wherein the proximal guidewire exit port is immediately proximal of the guider block.

16. Catheter as claimed in any one of claims 11 to 15, wherein the guider block is located around 75 cm from the distal tip of the catheter.

17. Catheter as claimed in claim 15 or 16, and including a steering tube (272) that protrudes from said exit port and can be pulled out of the catheter and wherein the guidewire lumen continues proximally from the exit port to a second more proximal exit port (224), proximal of the proximal exit port, and wherein the steering tube can be pulled out of the catheter to permit a guidewire to advance proximally beyond the location of the steering tube, as far as the more proximal exit port.

18. Catheter as claimed in claim 17, wherein the more proximal exit port is defined in a hub (224) that also defines a flushing port (222) for introduction of flushing fluid to the flushing lumen.

19. Catheter as claimed in any one of claims 1 to 18, including a self-expanding stent.

## Patentansprüche

1. Kathetervorrichtung mit einem Schaft, der sich von einem proximalen Ende zu einem distalen Ende erstreckt, um an seinem distalen Ende ein selbstexpandierendes Implantat (10) für intraluminales Vorrücken auf einem Führungsdraht (22) zu tragen und Abgabe des Implantats an einem Ort für einen Stent durch proximales Zurückziehen einer Umhüllung (14), die radial außerhalb des Implantats in dem Katheter liegt, wobei der Katheter ein erstes Schaftelement (32), um die Umhüllung proximal zu ziehen, und ein zweites Schaftelement (42, 52), um das Implantat distal zu drücken, um zu verhindern, dass das Implantat sich proximal mit der Hülle bewegt, wenn die Hülle proximal gezogen wird, aufweist, wobei
i) der Schaft ein Spüllumen definiert,
ii) der Schaft eine proximale Führungsdraht-Austrittsöffnung (20) definiert, die von dem proximalen Ende des Schafts distal ist, zum schnellen Austausch des Katheters in Bezug auf den Führungsdraht, und **dadurch gekennzeichnet, dass**:
iii) das erste Schaftelement ein Zugdraht ist,
iv) das zweite Schaftelement eine Schieberröhre (16) ist.

2. Katheter nach Anspruch 1, wobei die Umhüllung eine sich verjüngende distale Spitze (16) aufweist.

3. Katheter nach Anspruch 1 oder 2, wobei der Zugdraht durch einen inneren Zugring (28) an der Umhüllung angebunden ist, der radial im Inneren der Umhüllung angeordnet ist, wobei beide, der Zugdraht und der Zugring, aus Metall sind, und mit einer Metallbindung zwischen dem Zugdraht und dem Zugring.

4. Katheter nach Anspruch 3, mit einem äußeren Zugring (26), der radial außerhalb der Umhüllung und des inneren Zugrings ist, wobei die Umhüllung zwischen dem inneren und dem äußeren Zugring zusammengedrückt ist.

5. Katheter nach einem der vorhergehenden Ansprüche, wobei die Umhüllung polymer ist und in ihrer Wandstärke ein Geflecht aus metallischen Filamenten trägt.

6. Katheter nach einem der vorhergehenden Ansprüche, wobei die Schieberröhre (52) ein distales Ende aufweist, an dem Seite-an-Seite das proximale Ende einer Schieber-Leiter-Röhre (42) befestigt ist, die ein Lumen für den Führungsdraht definiert.

7. Katheter nach Anspruch 6, wobei die Schieber-Leiter-Röhre (42) aus einem spiralförmigen Metallfilament gebildet ist und einen Anschlagring (40) trägt, wobei die Schieberöhre, die Schieber-Leiter-Röhre und der Anschlagring als das zweite Schaftelement dienen.

8. Katheter nach Anspruch 7, wobei die Schieber-Leiter-Röhre sich distal des Stopprings zu der distalen Spitze des Katheters erstreckt.

9. Katheter nach Anspruch 6, 7 oder 8, mit einem Adapterblock (248), um die Schieberöhre und die Schieber-Leiter-Röhre zu verbinden, wobei der Block zwei Lumen Seite-an-Seite definiert, eines für die Schieberröhre und das andere für die Schieber-Leiter-Röhre.

10. Katheter nach einem der vorhergehenden Ansprüche, einschließlich einer Spülhülse (228), die mit der Umhüllung zusammenhängend ist und ein Lumen definiert, das die Schieberöhre enthält.

11. Katheter nach Anspruch 10, wobei der Schaft einen Leiterblock (230), der eine zylindrische Außenfläche aufweist, um die Spülhülse aufzunehmen, ein Führungsdrahtlumen und ein Lumen, Seite-an-Seite mit dem Führungsdrahtlumen, um die Schieberröhre zu empfangen, aufweist.

12. Katheter nach Anspruch 11, wobei die Spülhülse distal des Leiterblocks eine Balgröhre (232) aufweist.

13. Katheter nach Anspruch 11 oder 12, wobei die Spülhülse distal des Leiterblocks ein Teleskoprohr aufweist.

14. Katheter nach Anspruch 11, 12 oder 13, wobei der Leiterblock gegen eine axiale Bewegung in Bezug auf die Schieberröhre und in Bezug auf die Spülhülse auf seiner zylindrischen Oberfläche fixiert ist.

15. Katheter nach Anspruch 14, wobei die proximale Führungsdraht-Austrittsöffnung unmittelbar proximal des Leiterblocks ist.

16. Katheter nach einem der Ansprüche 11 bis 15, wobei der Leiterblock ca. 75 cm von der distalen Spitze des Katheters angeordnet ist.

17. Katheter nach Anspruch 15 oder 16, mit einer Steuerröhre (272), die von der Austrittsöffnung vorsteht, und aus dem Katheter herausgezogen werden kann und wobei das Führungsdrahtlumen sich proximal weiter von der Austrittsöffnung zu einer zweiten weiter proximalen Ausgangsöffnung (224) erstreckt, proximal der proximalen Austrittsöffnung, und wobei die Steuerröhre aus dem Katheter herausgezogen werden kann, um es einem Führungsdraht zu ermöglichen, proximal über die Position der Steuerröhre vorzurücken, so weit wie die weiter proximale Ausgangsöffnung.

18. Katheter nach Anspruch 17, wobei die weiter proximale Ausgangsöffnung in einer Nabe (224) definiert ist, die auch eine Spülöffnung (222) definiert, zum Einführen von Spülfluid zu dem Spüllumen.

19. Katheter nach einem der Ansprüche 1 bis 18, mit einem selbstexpandierenden Stent.

## Revendications

1. Dispositif cathéter comprenant une tige qui s'étend d'une extrémité proximale à une extrémité distale pour transporter au niveau de son extrémité distale un implant auto-extensible (10) pour une avancée intraluminale sur un fil guide (22) et la mise en place de l'implant au niveau d'un site d'implantation d'endoprothèse par le retrait proximal d'une gaine (14) qui repose radialement à l'extérieur de l'implant dans le cathéter, le cathéter comprenant un premier élément de tige (32) destiné à tirer la gaine de manière proximale et un second élément de tige (42, 52) destiné à pousser l'implant de manière distale pour empêcher que l'implant ne se déplace de manière proximale avec la gaine lorsque la gaine est tirée de manière proximale, dans lequel
i) la tige délimite une lumière de perfusion
ii) la tige délimite un orifice de sortie de fil guide proximal (20) qui est distal par rapport à l'extrémité proximale de la tige, pour un échange rapide du cathéter par rapport au fil guide, et **caractérisé en ce que** :
iii) le premier élément de tige est un fil de traction
iv) le second élément de tige est un tube de poussée (16).

2. Cathéter tel que revendiqué dans la revendication 1, dans lequel la gaine comporte une pointe distale conique (16).

3. Cathéter tel que revendiqué dans la revendication 1 ou 2, dans lequel le fil de traction est accouplé à la gaine par l'intermédiaire d'un anneau de traction interne (28) situé de manière radiale à l'intérieur de la gaine, le fil de traction et l'anneau de traction étant tous deux composés de métal et comprenant une liaison métallique entre le fil de traction et l'anneau de traction.

4. Cathéter tel que revendiqué dans la revendication 3, comprenant un anneau de traction externe (26) radialement à l'extérieur de la gaine et de l'anneau de traction interne, la gaine étant comprimée entre les anneaux de traction interne et externe.

5. Cathéter tel que revendiqué dans l'une quelconque des revendications précédentes, dans lequel la gaine est en matière polymère et transporte, à l'intérieur de l'épaisseur de sa paroi, une tresse de filaments métalliques.

6. Cathéter tel que revendiqué dans l'une quelconque des revendications précédentes, dans lequel le tube de poussée (52) présente une extrémité distale à laquelle a été fixée, à proximité immédiate, l'extrémité proximale d'un tube guide de poussée (42) qui délimite une lumière pour ledit fil guide.

7. Cathéter tel que revendiqué dans la revendication 6, dans lequel le tube guide de poussée (42) est formé d'un filament métallique en spirale et transporte un anneau d'étanchéité (40), le tube de poussée, le tube guide de poussée et l'anneau d'étanchéité servant en tant que lesdits seconds éléments de tige.

8. Cathéter tel que revendiqué dans la revendication 7, dans lequel le tube guide de poussée s'étend de manière distale par rapport à l'anneau d'étanchéité, jusqu'à la pointe distale du cathéter.

9. Cathéter tel que revendiqué dans la revendication 6, 7 ou 8, comprenant un bloc adaptateur (248) permettant de raccorder le tube de poussée et le tube guide de poussée, le bloc délimitant deux lumières situées à proximité immédiate l'une de l'autre, l'une pour le tube de poussée et l'autre pour le tube guide de poussée.

10. Cathéter tel que revendiqué dans l'une quelconque des revendications précédentes, comprenant un manchon de perfusion (228) qui est contigu à la gaine et qui délimite une lumière qui contient le tube de poussée.

11. Cathéter tel que revendiqué dans la revendication 10, dans lequel la tige comprend un bloc guide (230) qui comportent une surface extérieure cylindrique destinée à recevoir le manchonde perfusion, une lumière de fil guide, et une lumière située à proximité immédiate de la lumière du fil guide destinée à recevoir le tube de poussée.

12. Cathéter tel que revendiqué dans la revendication 11, dans lequel le manchon de perfusion, disposé de manière distale par rapport au bloc guide, comprend un tube de dilatation (232).

13. Cathéter tel que revendiqué dans la revendication 11 ou 12, dans lequel le manchon de perfusion, disposé de manière distale par rapport au bloc guide, comprend un tube télescopique.

14. Cathéter tel que revendiqué dans la revendication 11, 12 ou 13, dans lequel le bloc guide est fixé de sorte qu'il ne se déplace pas de manière axiale par rapport au tube de poussée et par rapport au manchon de perfusion sur sa surface cylindrique.

15. Cathéter tel que revendiqué dans la revendication 14, dans lequel l'orifice de sortie de fil guide proximal est immédiatement proximal du bloc guide.

16. Cathéter tel que revendiqué dans l'une quelconque des revendications 11 à 15, dans lequel le bloc guide est situé à environ 75 cm de la pointe distale du cathéter.

17. Cathéter tel que revendiqué dans la revendication 15 ou 16, et comprenant un tube de direction (272) qui fait saillie dudit orifice de sortie et qui peut être tiré du cathéter et dans lequel la lumière du fil guide continue de manière proximale à partir de l'orifice de sortie vers un second orifice de sortie (224) disposé de manière plus proximale, qui est proximal de l'orifice de sortie proximal, et dans lequel le tube de direction peut être tiré en dehors du cathéter pour permettre à un fil guide d'avancer de manière proximale au-delà de l'emplacement du tube de direction, jusqu'à l'orifice de sortie disposé de manière la plus proximale possible.

18. Cathéter tel que revendiqué dans la revendication 17, dans lequel l'orifice de sortie disposé de manière la plus proximale possible est défini dans un embout (224) qui définit également un orifice de perfusion (222) pour l'introduction du liquide de perfusion dans la lumière de perfusion.

19. Cathéter tel que revendiqué dans l'une quelconque des revendications 1 à 18, comprenant une endoprothèse auto-extensible.
